# EUROPEAN PATENT APPLICATION

(11) **EP 0 857 714 A1**
(43) Date of publication of application: **12.08.1998**
(21) Application number: 98200119.0
(22) Date of filing: 19.01.1998
(51) Int. Cl.: C07C 67/03, C07C 69/76

(54) **Recovery of monomers from polyethylene naphthalate**

(30) Priority: 31.01.1997 US 792239
(71) Applicant: EASTMAN KODAK COMPANY, Rochester, New York 14650 (US)
(72) Inventor: Naujokas, Andrius A., c/o Eastman Kodak Company, Rochester, New York 14650-2201 (US)
(74) Representative: Parent, Yves

(57) **Abstract**

There is described a process for depolymerizing polyethylene naphthalate polyester to monomer components which then can be used to make virgin polyester.

## Description

This invention relates to a process for recovering ester and glycol components from polyethylene naphthalate.

Polyethylene naphthalate, which also is called polyethylene 2,6 naphthalene dicarboxylate polyesters and is abbreviated as PEN, has outstanding physical properties for a number of uses, such as in textile, film and other applications. It has been found to be particularly useful as a film base for magnetic recording materials and the photographic industry has started to use it as a film support in new photographic products. PEN, as well as its precursor dimethyl 2,6 naphthalene dicarboxylate, abbreviated as NDC, is more costly than other polyesters, such as polyethylene terephthalate, that have been used in these products. The polymer is a linear polyester produced from ethylene glycol and dimethyl 2,6 naphthalene dicarboxylate (NDC) using conventional polymerization processes. Due to relatively high cost of the polyester and to minimize the need for disposal it is desirable to recycle and reuse waste PEN.

Two distinctly different routes have been used to recycle polyesters in general. The first route is direct recycle. This can be done when the polyester scrap is completely clean or can be cleaned by washing or other means. The clean(ed) polyester is used in place of virgin polyester at the appropriate point in the manufacturing process. Depending upon the manufacturing steps in the process, degradation of the polyester can occur during such steps as melting and extrusion due to thermal and shear effects. This results in a deterioration of the physical properties of the polyester, such as a lowering of molecular weight and formation of color. Generally for high grade products, such as film base, recycle polyester is blended in limited amounts with virgin polyester. Since PEN have relatively high melting and softening temperatures significant degradation of the polyester can be expected. Furthermore, if the polyester scrap is contaminated, or is not thoroughly cleaned, additional degradation of properties can occur.

The second route is depolymerization of the polyester to component monomers, recovering the monomers and then using them to produce new polyester. This route has been used for recovery of monomers from polyethylene terephthalate polyester, abbreviated as PET. Recovery techniques are described, for example in US-A-5,414,022; 4,163,860; 3,907,868; and 3,257,335.

PEN is a polycondensation polymer and, therefore, the molecular weight of the polymer can be reduced by reaction with a monohydric of dihydric alcohol at appropriate reaction conditions. The crude products then can be refined by methods such as distillation and crystallization to produce starting materials of suitable purity. Recovery of polyesters utilizes the chemistry of the polyester reaction. Synthesis and depolymerization paths are controlled by equilibrium considerations due to the reversibility of the reactions.

A linear polyester is prepared by reacting a difunctional organic acid and a difunctional alcohol. In some cases it is convenient to substitute the diester of the difunctional acid (for instance dimethyl 2,6 naphthalene dicarboxylate for the naphthalene 2,6 dicarboxylic acid). Usually the reaction is carried out at elevated temperature in the presence of an appropriate catalyst. Reaction by-products are removed from the reactor to drive the equilibrium in a direction that will cause the molecular weight of the polyester to increase.

In polyester scrap recovery, selected reaction by-products are added to the reactor in order to decrease the molecular weight of the polyester by depolymerization to monomer components. The diester form of recovered monomer is preferred, since the diester is more volatile than the acid and is more readily purified.

The generic polyester reaction can be written as two steps:
Step 1: Transesterification

   Dimethyl Ester + Glycol = Half Ester + Monomer + Methanol

   CH₃-A-CH₃ + HO-G-OH = CH₃-A-G-OH + HO-G-A-G-OH + CH₃-OH
Step 2: Polycondensation

   Monomer = Polymer + Glycol

   HO-G-A-G-OH = HO-(G-A)ₓ-G-OH + HO-G-OH
wherein:
A is a difunctional acid moiety (-COO-T-COO-)
T is benzene group for terephthalate and naphthalene group for NDC
G is an alkylene moiety (-CH₂-CH₂- for ethylene glycol)
x is the number of repeat units in an average molecular chain, that is, the degree of polymerization.

In the transesterification step the difunctional acid can be substituted for the diester and the reaction product will be water. Likewise other alcohols may be substituted for methanol to form the diester.

In polyester scrap recovery the above reactions are driven in the reverse direction, under appropriate reaction conditions and in the presence of catalysts, by addition of excess amounts of alcohol, glycol or both. Reacting the polymer with excess glycol will reverse the polycondensation reaction and will produce monomer (one diacid moiety with two glycol groups attached.) Other oligomers will be also present, as determined by the equilibrium conditions for the particular reaction. Reacting alcohol with monomer will then produce the difunctional ester product. Similar results will be obtained when excess alcohol (such as methanol) is reacted with the polymer. The final composition will again be governed by equilibrium. All the major components shown in the transesterification reaction, and a distribution of higher oligomers, are the reaction products.

The chemical steps used to depolymerized PEN are similar to any linear polyester. For instance excess glycol can be added and reacted with the scrap at elevated temperatures in the presence of appropriate catalysts to depolymerize the polyester to "monomer" or bis(2 hydroxyethyl 2,6 naphthalene dicarboxylate). Monomer has low volatility and cannot be purified by distillation. Washing and extraction are also difficult due to poor solids crystal structure. Sometimes purification can be achieved using an appropriate adsorbent. Generally it is difficult to purify monomer. An alternative depolymerization process consists of reacting the polyester with an alcohol such as methanol to produce the diester. Reaction of PEN with methanol will produce dimethyl (2,6 naphthalene dicarboxylate) (NDC). The diester has a relatively high melting point (about 190°C), however it can be distilled at reduced pressures. Contaminants generally present in the crude NDC as well as thermal degradation during distillation make it difficult to obtain high quality materials.

PEN can be depolymerized using methanol (or other monohydric alcohol) to the polyester constituents. Since methanol is a volatile material (BP 64°C) and the reaction must be carried out at elevated temperatures the reactor necessarily must be under pressure. Since all the components are in the reaction mass after depolymerization it is necessary to separate the various fractions to recover the desirable products. The reactor melt can be fractionated in an appropriate distillation train where methanol, glycol, NDC and the various contaminants can be separated. Typically a partial separation can be affected by reducing the temperature so that the NDC will crystallize leaving the liquid phase components in solution. The liquid phases then can be separated by filtration or other appropriate process. Further refining of NDC can be accomplished by washing the crystals. However, NDC generally forms small crystals that are difficult to filter and wash.

A need exists for a process so that PEN can be depolymerized using methanol and then crystallized forming readily filterable and washable NDC crystals and wherein the process can be continuous or batch.

The present invention provides a process for the depolymerizing of scrap polyethylene naphthalate polyester to monomer components that then can be used to make additional polyester. The process can be continuous or batch and the resulting crystals are readily filterable and washable.

In this process, scrap PEN is depolymerized in the presence of an ester exchange catalyst using a monohydric alcohol such as methanol at temperatures of 190°C (close to the melting point of NDC) to 290°C. In order to produce filterable and washable product of NDC, the crystallization step must be controlled through a cooling cycle and an appropriate amount of excess methanol used.

This process, and variations thereof, provide advantageous means for recovering monomer components from PEN polyester. The benefits and advantages of the processes will be apparent from the detailed discussion of the process below.

Although the polyester chemistry described above is well understood, implementing it in a practical recovery processes is not simple. Complex and sometimes interacting steps of heat and mass transfer must be considered. Some of the required processing steps include heat transfer, transport of two-phase or three-phase systems, crystallization, filtration, distillation, storage, as well as disposal of contaminants and reaction byproducts. Moreover, the physical properties of PEN and NDC differ markedly from PET and DMT. Therefore, experience in recovering DMT from PET is not directly translatable to recovery of NDC from PEN. For example, reactor design and process conditions required to recovery PEN will be different than those for PET. Physical properties of the polyester must be taken into consideration in determining suitable recovery conditions for PEN.

The process for depolymerization of PEN is pressurized alcoholysis. In this process polyethylene naphthalate polyester is depolymerized by alcoholizing the polyester with an excess of alcohol in a pressurized reactor at the vapor pressure of the alcohol at the temperature of the reaction.

In the high pressure alcoholysis process the reactants are at equilibrium at the end of the depolymerization step. The starting reactant composition determines the molecular weight distribution of the reaction products. Thus, excess alcohol is used to drive the reaction to the desired high concentration of monomer. Preferred alcohols are methanol or a combination of methanol and a mixture of alkylene glycols. Thus, the following discussion will be with respect to methanolysis, although it will be appreciated that other alcohols could be used with suitable modifications to the reaction conditions.

Methanol is a volatile material with a boiling point at ambient conditions of about 64°C. Operating the reactor at elevated pressure is done to obtain temperatures sufficiently high for the depolymerization reaction to proceed. Depolymerization of PEN can be accomplished either in a batch or continuous mode.

In a batch process solid PEN scrap is introduced into the reactor and an excess of methanol and a catalyst are added. Typical ratios of PEN and methanol are at least 2 moles of methanol per mole of PEN and preferably in the range of 2 to 20 moles of methanol per mole of PEN. The amount of catalyst used will vary depending upon the particular catalyst chosen, but typically is in the range of 20 to 100 parts by weight catalyst per million parts by weight of PEN. Suitable catalysts include Zn, Ca, Li, K and others. The reactor is then closed and the contents heated to the reaction temperature in the range of from 200 to 280°C and allowed to react until equilibrium is achieved. The pressure in the reactor will be a function the temperature. Typically, the reactor is operated at a pressure in the range of 2.5 x 10³ to 4.0 to 10³ Kpa. The reaction mixture is then cooled to a temperature of about 25 to 30°C, causing the diester to crystallize. It can be separated from the liquid phase, which contains unreacted methanol and glycols freed by the reaction. The products then can be refined to a desired degree of purity. The crystallization is controlled through a cooling cycle of preferably 0.5 to 5°C/min.

The continuous process differs from the batch process in that PEN scrap is first reduced in molecular weight by glycolysis to obtain a readily pumpable material. This can be accomplished by reacting the polymer with a glycol such as EG, DEG, TEG or others in a pressurized or atmospheric reactor. This material is then combined with methanol and a catalyst and introduced into a plug flow reactor designed to provide sufficient residence time for the reaction mixture to come to equilibrium. The proportions and operating conditions are similar as for the batch process.

The reactor conditions and compositions of the reaction mixtures are important in order to obtain a viable recovery process. The embodiment of the invention is illustrated using examples of a batch and a continuous recovery processes. The continuous reactor was simulated using process steps carried out batch wise. The actual continuous process can consist of one or more continuous stirred tank reactors, one or more plug flow reactors or a combination of the two types.

### Apparatus

The experiments were carried out using a 2 liter Parr reactor equipped with an agitator and pressure and temperature sensors. The reactor was heated electrically using external heating elements. The reactor temperature was regulated by a controller.

### Procedure

Ground PEN scrap was introduced into the reactor and methanol and catalyst added in the desired proportions. The reactor was then closed and the heating cycle started with agitation. When the reactor contents reached 190°C the timer was started. The reaction was then allowed to proceed for the chosen time interval. At the end of the run the whole reactor assembly was immersed in cold water to cool reaction products and to reduce the internal pressure. At the end of the cooling period the reactor was disassembled and the reaction products removed for further processing, analysis and evaluation. When slow crystallization rate was desired the reactor was cooled by allowing heat loss to the surroundings.

### Example 1

### Batch Reactor

Both fast and slow crystallization rates were evaluated in the batch mode. When the reactor mixture was cooled rapidly by immersing the reactor assembly in cold water ANDC crystal slurry was fonned that was difficult to filter and wash. At higher methanol charge, however, improved crystals were obtained. Table 1 lists the reaction conditions and the results of the experimental runs. The NDC concentrations are for methanol washed samples. The crystal quality was estimated from filtration rates.

**Table 1**

| Pressure Reactor Methanolysis | | | | | | | |
|---|---|---|---|---|---|---|---|
| Run No. | Time hr. | Temp. C | PEN g | Methanol g | Cooling Cycle | Crystal Quality | NDC % |
| 1 | 4 | 189 | 214 | 210 | Slow | Good | 88 |
| 2 | 4 | 186 | 214 | 410 | Fast | Good | 91 |
| 3 | 4 | 205 | 214 | 420 | Slow | Good | 92 |
| 4 | 6 | 190 | 214 | 210 | Slow | Good | 98 |
| 5 | 4 | 200 | 214 | 210 | Fast | Poor | 84 |

The reaction occurred at relatively low temperatures. It can be seen in Table 1 that PEN was depolymerized close to the melting point of NDC. At 420 g methanol charge faster cooling rate still produced adequate crystal size distribution.

### Example 2

### Simulation of Continuous Reactor

For a continuous reactor operation the reactor feed must be rendered pumpable. The feed scrap also can be melted in an extruder and then introduced into the methanolysis reactor. A more practical method consists of reducing the feed viscosity through a glycolysis step. To simulate a continuous operation first the PEN scrap was reduced in a molecular weight using a mixture of high boiling glycols. The mixture by weight consisted of the following:
Diethylene Glycol 63%
Triethy]ene Glycol 33%
Cyclohexane Dimethanol 4%

Due to relatively low boiling point of ethylene glycol ambient pressure glycolysis with this material is not practical. A pressurized reactor is needed to glycolize PEN with ethylene glycol.

The reduced molecular material was then placed into the Parr reactor and the required amount of methanol added. The depolymerization then was carried out as indicated before.

The results are tabulated in Table 2. The reaction times are in hours and the feed to the pressurized methanolysis step consists of the material produced in the glycolysis step. In all case

**Table 2**

| Ambient Pressure Glycolysis | | | | |
|---|---|---|---|---|
| Run | Time Hrs. | Temp. °C | Glycols, g | Scrap, g |
| 1 | 4 | 236 | 200 | 428 |
| 2 | 4 | 240 | 200 | 428 |
| 3 | 3 | 236 | 200 | 428 |
| Catalyst - ZnAC 0.8g | | | | |

| Pressurized methanolysis | | | | | |
|---|---|---|---|---|---|
| Run | Time | Temp. °C | Feed, g | Methanol | NDC |
| 1 | 2 | 200 | 200 | 421 | 87 |
| 2 | 4 | 200 | 200 | 421 | 86 |
| 3 | 8 | 200 | 200 | 421 | 85 |
| 4 | 6 | 205 | 200 | 421 | 86 |
| Catalyst - ZnAC 0.8 g | | | | | |

In all cases the methanolyzed material was crystallized using a slow cycle and filterable slurries were obtained.

It is established that not only the conversion but also the quality of the NDC crystals must be obtained to get a viable recovery process. The crystal quality was measured by filtration. In the cases where poor crystal quality was obtained the solids were difficult to filter and wash.

### Discussion

Kinetically the effect of glycols is detrimental on the methanolysis reaction since due to reaction the glycol and the ester tend to form monomers and half esters. The mass transfer rates could be increased, however, by forming more soluble intermediates.

The glycols concentrations used in the experiments are generally equamolar to the scrap at the 100 gram "sludge" runs and 0.5:1 for the 50 gram "sludge" run. Since each polyester repeat unit has one molecule of glycol equimolar concentration is stoichiometrically equivalent to a monomer. The methanol concentration is in a molar methanol to scrap polyester ratio of 7.4.

The stoichiometric methanol mole ratio for polyesters is two to one. This means that two molecules of methanol are required to convert each polymer repeat unit (Mw 241) to NDC. Generally higher methanol to polymer ratios must be used to obtain high conversions. In the case of PEN with the reaction temperature close to the melting point of NDC larger excess of methanol may be beneficial. Methanol is a reactant and also acts as a solvent for the NDC produced by reaction. In order to promote the PEN depolymerization reactions, relatively high mole ratios were used. For the 210 gram of methanol runs the methanol to scrap ratio was 7.4 and for 420 grams of methanol mole ratio of 14.8 resulting in fourfold and eight fold excess respectively. It appears that the mole ratio of 7.4 is adequate to obtain the needed conversion. There is a possibility that the mole ratio can be further reduced.

From the above results it is evident that mixed reactant processes do not give a significant advantage over the simple methanolysis process of PEN. For a batch process single reactant methanolysis can be used. For a continuous methanolysis process, however, the molecular weight of the feed must be reduced to obtain pumpable material at reasonable temperatures.

## Claims

1. A process for recovering monomer components from polyethylene naphthalate polyester, the process comprising the steps of:
a) contacting polyethylene naphthalate polyester with a mono-and/or di.-hydric alcohol at a temperature and pressure sufficient to cause the alcohol to depolymerize the polyester, and
b) separating from one another, in any order, the alcohol, the monomer components and the reaction residue wherein polyethylene naphthalate polyester is depolymerized by a pressure alcoholysis process comprising the step of alcoholizing the polyester with an excess of alcohol in a reactor at a pressure of reaction at the vapor pressure of the alcohol at the reaction temperature at mole ratio of alcohol to polymer is at least 2 at a temperature of from 190° to 290°C.

2. The process of claim 1, wherein the mole ratio of alcohol to polymer is from 7 to 20.

3. The process of claim 1, wherein the alcohol is methanol.

4. The process of claim 1, wherein the alcohol is a combination of methanol and a mixture of alkylene glycols.

5. The process of claim 1, wherein the starting proportions of alcohol to polyester are 10 parts by weight alcohol per 100 parts by weight polyester.

6. The process of claim 1, wherein the steps are carried out in batch.

7. The process of claim 1, wherein the reactions are carried out continuously.

8. The method of claim 7, wherein the reactants are first melted in an extruder and then introduced into methanolysis reactor.

9. The method of claim 7, wherein the polyethylene naphthalate scrap is first reduced in molecular weight using a mixture of high boiling glycols.

10. The process of claim 9, wherein the high boiling glycols are diethylene glycol, triethylene glycol and cyclohexane dimethanol.
